# EUROPEAN PATENT SPECIFICATION

(11) **EP 0 793 964 B1**
(45) Date of publication and mention of the grant of the patent: **16.12.1998**
(21) Application number: 96301500.3
(22) Date of filing: 05.03.1996
(51) Int. Cl.: A61K 35/78

(54) **Compositions for treatment of Hepatitis C**
Zubereitungen für die Behandlung von Hepatitis C
Compositions pour le traitement de l'hépatite C

(43) Date of publication of application: 10.09.1997
(73) Proprietor: BIO-PHYSIO PHARMACEUTICAL RESEARCH AND DEVELOPMENT COMPANY LIMITED, Valletta (MT)
(72) Inventor: Abd El-Baset Mohamed Sayed Abd El-Aziz, Dokki, Cairo (EG); Amira Sayed Ramadan, Dokki, Cairo (EG)
(74) Representative: Goldin, Douglas Michael

(56) References cited:
- DE-A- 2 459 450
- PLANTES MEDICINALES ET PHYTOTHERAPIE, vol. 16, no. 4, October 1982, pages 260-279, XP000579811 K. BOUKEF ET AL.: "CONTRIBUTION A L'ETUDE DES PLANTES UTILISEES EN MEDECINE TRADITIONELLE TUNISIENNE"
- PATENT ABSTRACTS OF JAPAN vol. 009, no. 119 (C-282), 23 May 1985 & JP-A-60 009481 (KANETO YOSHIDA), 18 January 1985,

## Description

The present invention relates to the treatment of Hepatitis C.

Five different viruses, denoted by the letters A, B, C, D and E, cause viral Hepatitis. Hepatitis C is known to account for the great majority of what was previously referred to as non-B Hepatitis. Chronic Hepatitis C is a slowly progressive disease which gradually advances over 10 to 40 years. An individual infected with HCV is also likely to have an increased chance of developing hepatocellular carcinoma, a type of primary liver cancer. The Hepatitis C virus (HCV) was identified and described in 1989. In 1990 a Hepatitis C antibody test became commercially available to help identify individuals exposed to HCV.

Individuals infected with HCV are often identified because they are found to have elevated levels of liver enzymes on routine blood tests or because Hepatitis C antibodies are found following blood donation. In 1992 a more specific test for Hepatitis C antibodies became available which eliminated some of the false positive results which previously occurred. In general, elevated levels of liver enzymes and a positive antibody test for HCV are now taken to indicate that an individual has chronic Hepatitis C.

It is known that the juice of the fruit *Ecballium elaterium* and that the juice of the fruit *Paris incompleta* will, separately, act against jaundice (Dawod. O. El-Antaki, (1596), Recipe of Dawod El-Antaki, 1st part, page 81). It is also known in traditional Tunisian medicine that preparations containing *Ecballium elatrium* can be used for the treatment of Hepatitis and sinusitis (Boukef *et al*. Plantes Médicinales et Phytothérapie, vol. 16, no. 4, 1982, pages 260-279) however no further information is available from that reference.

The applicant has surprisingly discovered that Hepatitis C may be treated by individual extracts from wild plants, and that a certain combination of extracts provides a synergistic effect.

Accordingly the present invention provides a composition comprising:
i) the detoxified juice of the fruit of *Ecballium elaterium*, and
ii) the detoxified juice of the fruit of *Paris* *incompleta*,
   wherein the volume ratio of the juice of the fruit of *Ecballium elaterium* to the juice of the fruit of *Paris incompleta* is from 10:1 to 2:1.

The present invention also provides a combination of (i) the detoxified juice of the fruit *Ecballium elaterium*, and (ii) the detoxified juice of the fruit of *Paris incompleta*, wherein the volume ratio of the juice of the fruit of *Ecballium elaterium* to the juice of the fruit of *Paris incompleta* is from 10:1 to 2:1, for use in a method of treatment of the human or animal body by therapy, in particular for use in a method of treatment of Hepatitis C.

The present invention further provides the use of the detoxified juice of the fruit of *Ecballium elaterium* in the preparation of a medicament for the treatment of Hepatitis C.

The present invention additionally provides the use of the detoxified juice of the fruit of *Paris incompleta* in the preparation of a medicament for the treatment of Hepatitis C.

The present invention also provides a process for preparing a composition as defined above which comprises:
(i) separately crushing the fruits of *Ecballium elaterium* and *Paris incompleta*, filtering the juices thus obtained to remove toxins and mixing the juices together; or
(ii) separately crushing the fruits of *Ecballium elaterium* and *Paris incompleta*, mixing the juices thus obtained together and filtering the combined juices to remove toxins; or
(iii) crushing the fruits of *Ecballium elaterium* and *Paris incompleta* together and filtering the juice to remove toxins.

The juices of the fruits may be obtained simply by crushing the fruits. The juices can be detoxified by any means, for example by separating out toxins by filtration. By "detoxified" is meant removal of undesired components which may, for example, have a laxative effect.

The detoxified juice of the fruit *Ecballium elaterium* and the detoxified juice of the fruit *Paris* *incompleta* each have, separately, an effect against Hepatitis C. Thus each juice reduces the level of bilirubin in a subject and reduces the activity of SGOT, SGPT and alkaline phosphatase. A combination of the juices may be used, to be administered separately or together in a single composition. If both juices are used, they may be used in any relative amounts. However, it has been found that a synergistic effect, particularly in the reduction of the level of bilirubin or the reduction in activity of SGOT, SGPT and/or alkaline phosphatase, may be obtained if the juices are administered in particular volume ratios. A synergistic effect can be obtained if the volume ratio of the juice of the fruit of *Ecballium elaterium* to the juice of the fruit of *Paris incompleta* is from 10:1 to 2:1, preferably from 8:1 to 2:1, more preferably from 5:1 to 3.5:1, most preferably about 4:1.

The composition, or each juice separately, may be diluted with a suitable diluent, for example water. Desirably the composition contains from 30 to 70 volume% of the juice or combination of juices, preferably from 40 to 60% more preferably about 50%. The composition may contain further components which are known to be effective in the treatment of Hepatitis C or related diseases.

According to the present invention the composition or juice may be administered by any suitable route to a subject in need of treatment. The two juices can be administered together or separately. The order of administration of the two juices is not significant.

The composition or juices of the present invention may be administered by any suitable route of administration. Desirably the composition or juices are administered nasally in the form of nasal drops or are administered as suppositories. It may also be administered as sublingual drops, although this route of administration is less effective.

The dose for administration varies between wide limits. Generally for humans the dose may vary from 0.15 to 0.5ml of mixture of juices (calculated as pure juices) per day, preferably from 0.2 to 0.3ml for administration as nasal drops. Desirably the drops are administered twice per day, and in each nasal passage. For suppositories, the doses may vary from 0.3 to 0.8ml of juices (calculated as pure juices) per day, preferably from 0.4 to 0.6ml. Desirably the suppositories are administered twice per day. For pure juices the doses may vary from 0.1 to 0.2ml, for *Ecballium elaterium* and from 0.05 to 0.1ml for *Paris incompleta*. The treatment is generally continued for from 45 to 60 days after HCV-RNA in the serum is no longer detectable, and a maintenance dose should continue until HCV-RNA in peripheral leucocytes is also negative, as determined by PCR. Maintenance doses may be required, for example, for a further 2 to 4 months. The maintenance dose may be, for example, one drop in each nasal passage daily. It is preferable to confirm complete recovery using serotyping assays, when these are commercially available.

The treatment may be carried out together with other treatments of Hepatitis C. For example herbs or mixtures of herbs which are known to act as liver protecting agents may also be administered at the same time or before or after administration of the juices. Such herbs include *Phyrynlum emblica*, *Negila sativa*, *Solenostemma argel*, *Zyzyphus spina christi*, *Berberis vulgaris*, *Alhagi maurorum*, *Clome africana*, *Sesbamia aculeata*, *Peganum harmala*, *Rheum officinale*, *Erythraea centaurium* and *Fragaria vesca*. Desirably the herbal mixture is a mixture of all of the above herbs. The mixture of herbs may be administered orally, for example in a dose of 5g three times a day. Maintenance doses of the herbs may also be administered with the maintenance doses of the compositions of the present invention. Suitable maintenance doses of the mixture of herbs are 3 to 5g twice a day.

The present invention is further described in the following Examples:

### EXAMPLE 1

### Preparation of a Nasal Drop Composition

The fruit of *Ecballium elaterium* and the fruit of *Paris incompleta* were separately crushed. The juices of each fruit were diluted with distilled water in a volume ratio of 1:0.5. The turbidity of each extract was separated by filtration using a Wattman No. 4 filter. The juices were then mixed in amounts of 79.5 parts by volume *Ecballium elaterium* juice and 19.5 parts by volume *Paris incompleta* juice and the pH was adjusted to 7 using sodium carbonate. The final extract was sterilized using a Millipore 0.45µm filter. The sterilized extract was then formulated in a known manner into 0.06ml nasal drops.

### EXAMPLE 2

### Toxicity Test

The toxicity of the combined extract obtained in Example 1, before being formulated into nasal drops, was tested using albino mice. 2ml of the extract was administered orally to a group of 60 mice morning and evening (a total of 4ml) for 45 days. The liver and kidney functions and blood composition were determined before the treatment started, after each of the first 4 weeks of treatment and after 2 months. Determination was carried out by obtaining blood samples following decapitation of a group of 10 mice for each measurement. The results are shown in the following Table 1:

**Table 1**

| Subject | Total protein | Albumin | SGOT | SGPT | Urea | Creatinine |
|---|---|---|---|---|---|---|
| Control | 6.5±0.01 | 3.3±0.01 | 26±1.8 | 23±1.6 | 14+1.1 | 0.6±0.02 |
| After 1st week | 6.6±0.01 | 3.4±0.01 | 25±1.2 | 22±1.3 | 14±1.0 | 0.65±0.01 |
| After 2nd week | 6.6±0.01 | 3.5±0.01 | 23±1.5 | 20±1.0 | 13±1.0 | 0.62±0.01 |
| After 3rd week | 6.7±0.01 | 3.5±0.01 | 22±1.5 | 20±1.0 | 12±1.0 | 0.6±0.02 |
| After 4th week | 6.7±0.02 | 3.5±0.01 | 20±1.0 | 18±1.0 | 12±2.0 | 0.6±0.02 |
| After 2 months | 6.8±0.03 | 3.6±0.02 | 20±1.0 | 18±1.2 | 13±2.0 | 0.65±0.04 |

The results showed that the combined extract markedly reduces liver transaminases and increases the albumin concentration. The extract does not affect the creatinine and urea levels, nor does it affect the composition of the blood. No diarrhoea or constipation in the mice was observed.

### EXAMPLE 3

### In Vivo Treatment

The nasal drops obtained in Example 1 were administered to a selection of human patients.

Four groups of patients and a control groups were used as follows:
- Group 1:: Patients recently discovered as hepatitis C viraemia. HCV, Riba, antibody and HCV-RNA as determined by PCR were positive. Impaired liver function was observed.
- Group 2:: Patients having chronic active hepatitis with cirrhotic livers, secondary to hepatitis C virus infection, with and without ascites. This group had previously been given a full course of interferon therapy (3 million units) on alternate days for 4 months, and then alpha interferon (5 million units) every other day for 5 months. After this treatment investigations showed that the transaminase levels were still markedly elevated, the albumin levels ranged from 2.5 to 4.8, the platelet levels ranged from 50,000 to 85,000 and the HCV-RNA was positive.
- Group 3:: Patients having active cirrhosis with hepatosplenomegaly with and without ascites. This group had previously been treated with alpha interferon (3 million units) every other day coupled with ribaaverin for 6 months. After this treatment investigations showed that the transaminase levels were still high and the HCV-RNA was positive, as determined by PCR.
- Group 4:: Patients who required 3 to 4 months for recovery. This group represents the more tolerant viremia.

Each of the above groups, and the control group, were treated by administration of the nasal drops obtained in Example 1 at a dose per subject of 1 drop in each nasal passage morning and evening. The treatment was continued for from 4 to 8 weeks for each patient. The duration was dependant on the bilirubin levels in the serum of each patient.

The results are shown in the following Table 2:

**Table 2**

| BEFORE TREATMENT | | | | | | |
|---|---|---|---|---|---|---|
| | Liver function | | | | | |
| | Total Bilirubin Direct | | Alb. | SGOT | SGPT | PCR |
| Control N = 10 | 0.5±0.01 | 0.1±0.01 | 4.1±0.1 | 23±2 | 21±1.5 | -ve |
| 1st group* N = 42 | 1.03±0.5 | 0.3±0. | 3.8±0.1 | 71±5 | 64±4 | +ve |
| 2nd group** N = 42 | 3.2±0.8 | 1.8±0. | 2.8±0.3 | 170±14 | 164±10 | +ve |
| 3rd group*** N = 15 | 3.0±1.9 | 2±0. | 2.9±0.2 | 260±16 | 285±18 | +ve |
| 4th group**** N = 15 | 1.9±0.2 | 0.4±0. | 3.7±0.4 | 140±9 | 112±12 | +ve |

| AFTER TREATMENT (6-9 months later) | | | | | | |
|---|---|---|---|---|---|---|
| Control N = 10 | 0.4±0.02 | 0.1±0.0 | 4.2±0.1 | 20±0.1 | 18±1.0 | -ve |
| 1st group* N = 42 | 0.6±0.1 | 0.2±0. | 4.0±0.1 | 32±1.8 | 35±2.1 | -ve |
| 2nd group** N = 42 | 0.7±0.2 | 0.2±0.5 | 3.8±0.1 | 46±2 | 49±1.5 | -ve |
| 3rd group*** N = 15 | 0.9±0.3 | 0.3±0. | 4.2±0.2 | 50±4 | 62±5.0 | -ve |
| 4th group**** N = 15 | 0.9±0.2 | 0.3±0. | 3.9±0.1 | 52±3 | 59±4 | -ve |

| | | | | | | |
|---|---|---|---|---|---|---|
| * 34 patients responded to treatment out of 42 | | | | | | |
| ** 23 patients responded to treatment out of 42 (need 2 months) | | | | | | |
| *** 12 patients responded to treatment out of 15 | | | | | | |
| **** 10 patients responded to treatment out of 15 (need 3-4 months) | | | | | | |

A gradual but marked decrease in the Bilirubin, SGOT, and SGPT values was noted. Moreover all patients mentioned that their physical activity was improved and that less effort for movement was required. For example a patient initially having values as follows:

| | |
|---|---|
| SGPT | 930 U/l |
| SGOT | 363 U/l |
| γGT | 488 |
| albumin | 2.98 |
| total bilirubin | 3.3 mg/dl |
| direct bilirubin | 204 mg/dl; |

had the following values two weeks later:

| | |
|---|---|
| SGPT | 334 U/l |
| SGOT | 220 U/l |
| γGT | 280 |
| albumin | 3.2 |
| total bilirubin | 0.45 mg/dl |
| direct bilirubin | 0.15 mg/dl |

Finally both serum HCV-RNA by PCR and HCV-RNA in peripheral leucocytes were negative.

Two patients who suffered from chronic viral end-stage liver disease were advised to have orthoptic liver transplants. However, following the abovementioned treatment the liver functions became normal. Thus the albumin level became normal and HCV-RNA was not detected by PCR assay.

In summary, 79 out of the 114 patients tested had a complete recovery. Only 6 patients still tested positive for HCV-RNA as determined by PCR, but nevertheless had improvements in their liver functions.

Follow up of these 79 patients by determination of HCV-RNA 6 to 9 months after treatment showed a marked recovery and stable levels of the compounds referred to in Table 2.

### EXAMPLE 4

### Treatment with extracts from Ecballium elaterium or Paris incompleta alone.

The fruit of *Ecballium elaterium* and the fruit of *Paris incompleta* were separately crushed. The juices of each fruit were diluted with distilled water in a volume ratio of 1:0.5. The turbidity of each extract was separated by filtration using a Wattman No. 4 filter. The pH of each composition was adjusted to 7 using sodium carbonate. Each final extract was sterilized using a Millipore 0.45µm filter. Each sterilized extract was then formulated in a known manner into 0.06ml nasal drops.

These nasal drops were administered to a selection of human patients at a dose of 1 drop in each nasal passage morning and evening. The treatments were continued for from 4 to 8 weeks for each patient. The duration was dependant on the bilirubin levels in the serum of each patient.

The results were as follows:

| Effect of nasal drops of detoxified juice of the fruit of *Ecballium elaterium* and mixture of herbs on hepatitis-C viraemic patients. BEFORE TREATMENT | | | | | | |
|---|---|---|---|---|---|---|
| | Bilirubin | | Alb. | SGOT | SGPT | PCR |
| | Total | Direct | | | | |
| N = 50* | 3.8±0.1 | 2.6±0.1 | 2.3±0.1 | 235±32 | 260±38 | (+) |

| AFTER TREATMENT | | | | | | |
|---|---|---|---|---|---|---|
| N = 50* | 0.6±0.1 | 0.2±0.02 | 4.2±0.2 | 45±4 | 48±5 | (-) |

| | | | | | | |
|---|---|---|---|---|---|---|
| * No. of responded patients 31 from 50 | | | | | | |

| Effect of nasal drops of detoxified juice of the fruit of *Paris incompleta* and mixture of herbs on hepatitis-C viraemic patients. BEFORE TREATMENT | | | | | | |
|---|---|---|---|---|---|---|
| | Bilirubin | | Alb. | SGOT | SGPT | PCR |
| | Total | Direct | | | | |
| N = 50** | 3.1±0.2 | 2.5±0.1 | 3.0±0.1 | 210±25 | 220±30 | (+) |

| AFTER TREATMENT | | | | | | |
|---|---|---|---|---|---|---|
| N = 50** | 0.9±0.2 | 0.4±0.02 | 3.6±0.2 | 52±5 | 58±6 | (-) |

| | | | | | | |
|---|---|---|---|---|---|---|
| ** No. of responded patients 26 from 50 | | | | | | |

## Claims

1. A composition comprising:
(i) the detoxified juice of the fruit of *Ecballium elaterium*; and
(ii) the detoxified juice of the fruit of *Paris incompleta*,
wherein the volume ratio of the juice of the fruit of *Ecballium elaterium* to the juice of the fruit of *Paris incompleta* is from 10:1 to 2:1.

2. A composition according to claim 1 in which the ratio is about 4:1.

3. A composition according to claim 1 or 2 which further comprises water.

4. A process for preparing a composition as defined in any one of the preceding claims which comprises:
(i) separately crushing the fruits of *Ecballium elaterium* and *Paris incompleta*, filtering the juices thus obtained to remove toxins and mixing the juices together; or
(ii) separately crushing the fruits of *Ecballium elaterium* and *Paris incompleta*, mixing the juices thus obtained together and filtering the combined juices to remove toxins; or
(iii) crushing the fruits of *Ecballium elaterium* and *Paris incompleta* together and filtering the juice to remove toxins.

5. A combination of (i) the detoxified juice of the fruit of *Ecballium elaterium*, and (ii) the detoxified juice of the fruit of *Paris incompleta*, wherein the volume ratio of the juice of the fruit of *Ecballium elaterium* to the juice of the fruit of *Paris incompleta* is from 10:1 to 2:1, for use in a method of treatment of the human or animal body by therapy.

6. A combination according to claim 5 for use in a method of treatment of Hepatitis C.

7. A composition according to any one of claims 1 to 3 for use in a method of treatment of the human or animal body.

8. Use of the detoxified juice of the fruit of *Ecballium elaterium* in the preparation of a medicament for the treatment of Hepatitis C.

9. Use of the detoxified juice of the fruit of *Paris incompleta* in the preparation of a medicament for the treatment of Hepatitis C.

10. Use of a composition as defined in any one of claims 1 to 3 in the preparation of a medicament for the treatment of Hepatitis C.

## Patentansprüche

1. Zusammensetzung, umfassend:
(i) den entgifteten Saft der Frucht von *Ecballium elaterium*; und
(ii) den entgifteten Saft der Frucht von *Paris incompleta*,
wobei das Volumenverhältnis des Saftes der Frucht von *Ecballium elaterium* zu dem Saft der Frucht von *Paris incompleta* von 10:1 bis 2:1 beträgt.

2. Zusammensetzung gemäß Anspruch 1, in welcher das Verhältnis etwa 4:1 ist.

3. Zusammensetzung gemäß Anspruch 1 oder 2, welche außerdem Wasser enthält.

4. Verfahren zur Herstellung einer Zusammensetzung wie in einem beliebigen der vorausstehenden Ansprüche definiert, welches umfaßt:
(i) das getrennte Zerkleinern der Früchte von *Ecballium elaterium* und *Paris incompleta*, das Filtern der so erhaltenen Säfte zur Entfernung von Toxinen und das Zusammenmischen der Säfte; oder
(ii) das getrennte Zerkleinern der Früchte von *Ecballium elaterium* und *Paris incompleta*, das Zusammenmischen der so erhaltenen Säfte und das Filtern der vereinigten Safte, um Toxine zu entfernen; oder
(iii) das gemeinsame Zerkleinern der Früchte von *Ecballium elaterium* und *Paris incompleta* und das Filtern des Saftes, um Toxine zu entfernen.

5. Kombination von (i) dem entgifteten Saft der Frucht von *Ecballium elaterium* und (ii) dem entgifteten Saft der Frucht von *Paris incompleta*, worin das Volumenverhältnis des Saftes der Frucht von *Ecballium elaterium* zu dem Saft der Frucht von *Paris incompleta* von 10:1 bis 2:1 beträgt, zur Verwendung in einem Behandlungsverfahren für den menschlichen oder tierischen Körper durch eine Therapie.

6. Kombination gemäß Anspruch 5 zur Verwendung in einem Behandlungsverfahren für Hepatitis C.

7. Zusammensetzung gemäß einem beliebigen der Ansprüche 1 bis 3 zur Verwendung in einem Behandlungsverfahren für den menschlichen oder tierischen Körper.

8. Verwendung des entgifteten Saftes der Frucht von *Ecballium elaterium* bei der Herstellung eines Medikamentes für die Behandlung von Hepatitis C.

9. Verwendung des entgifteten Saftes der Frucht von *Paris incompleta* bei der Herstellung eines Medikaments für die Behandlung von Hepatitis C.

10. Verwendung einer Zusammensetzung wie in einem beliebigen der Ansprüche 1 bis 3 definiert bei der Herstellung eines Medikaments für die Behandlung von Hepatitis C.

## Revendications

1. Composition comprenant :
(i) le jus détoxifié du fruit *Ecballium elaterium* ; et
(ii) le jus détoxifié du fruit *Paris incompleta*,
dans laquelle le rapport en volume du jus du fruit *Ecballium elaterium* au jus du fruit *Paris incompleta* est de 10:1 à 2:1.

2. Composition selon la revendication 1, dans laquelle le rapport est d'environ 4:1.

3. Composition selon la revendication 1 ou 2, qui comprend en outre de l'eau.

4. Procédé de préparation d'une composition telle que revendiquée selon l'une quelconque des revendications précédentes, qui comprend les étapes consistant :
(i) à écraser séparément les fruits de *Ecballium elaterium* et *Paris incompleta*, à filtrer les jus ainsi obtenus pour en éliminer les toxines et à mélanger les jus entre eux ; ou
(ii) à écraser séparément les fruits de *Ecballium elaterium* et *Paris incompleta*, à mélanger les jus entre eux et à filtrer les jus réunis pour en éliminer les toxines ; ou
(iii) à écraser ensemble les fruits de *Ecballium elaterium* et *Paris incompleta*, et à filtrer le jus pour en éliminer les toxines.

5. Combinaison (i) du jus détoxifié du fruit *Ecballium elaterium* et (ii) du jus détoxifié du fruit *Paris incompleta*, dans laquelle le rapport en volume du jus du fruit *Ecballium elaterium* au jus du fruit *Paris incompleta* est de 10:1 à 2:1, destinée à être utilisée dans un procédé de traitement thérapeutique du corps humain ou animal.

6. Combinaison selon la revendication 5, destinée à être utilisée dans un procédé de traitement de l'hépatite C.

7. Composition selon l'une quelconque des revendications 1 à 3, destinée à être utilisée dans un procédé de traitement du corps humain ou animal.

8. Utilisation du jus détoxifié du fruit *Ecballium elaterium* dans la préparation d'un médicament destiné au traitement de l'hépatite C.

9. Utilisation du jus détoxifié du fruit *Paris incompleta* dans la préparation d'un médicament destiné au traitement de l'hépatite C.

10. Utilisation d'une composition telle que revendiquée selon l'une quelconque des revendications 1 à 3, dans la préparation d'un médicament destiné au traitement de l'hépatite C.
